# EUROPEAN PATENT APPLICATION

(11) **EP 4 230 723 A1**
(43) Date of publication of application: **23.08.2023**
(21) Application number: 21879173.9
(22) Date of filing: 09.09.2021
(51) Int. Cl.: C12N 1/21, C12N 1/20, C12N 9/12, C12N 15/54, C12N 15/70, C12P 13/08

(54) **POLYPEPTIDE WITH ASPARTATE KINASE ACTIVITY AND USE THEREOF IN PRODUCTION OF AMINO ACID**

(30) Priority: 16.10.2020 CN 202011110184
(71) Applicant: Tianjin Institute of Industrial Biotechnology, Chinese Academy of Sciences, Tianjin Airport Economic Area Tianjin 300308 (CN)
(72) Inventor: ZHENG, Ping, Tianjin 300308 (CN); LIU, Jiao, Tianjin 300308 (CN); WANG, Yu, Tianjin 300308 (CN); ZHOU, Wenjuan, Tianjin 300308 (CN); SUN, Jibin, Tianjin 300308 (CN); CHEN, Jiuzhou, Tianjin 300308 (CN); MA, Yanhe, Tianjin 300308 (CN)
(74) Representative: Weickmann & Weickmann PartmbB
(86) International application number: PCT/CN2021/117492
(87) International publication number: WO 2022/078127

(57) **Abstract**

Provided are a polypeptide with aspartate kinase activity and the use thereof in the production of an amino acid. Specifically, provided are a novel polypeptide with aspartate kinase activity, a recombinant polypeptide, a polynucleotide, a nucleic acid construct, a recombinant expression vector, a recombinant host cell, and a method for producing an amino acid. The polypeptide with aspartate kinase activity is a mutant mutated at one or more positions corresponding to positions 293, 294 and 307 of the amino acid sequence as shown in SEQ ID NO: 1. Compared with a polypeptide having the sequence as shown in SEQ ID NO: 1, the mutant polypeptide removes the feedback inhibition of lysine on aspartate kinase, has high aspartate kinase activity, and can be used for the stable and efficient production of lysine and derivatives thereof.

## Description

### TECHNICAL FIELD

The present disclosure relates to the field of molecular biology and bioengineering, in particular to a polypeptide with aspartate kinase activity, a recombinant polypeptide, a polynucleotide encoding the polypeptide or the recombinant polypeptide, a nucleic acid construct, a recombinant expression vector, a recombinant host cell, and a method for producing an amino acid.

### BACKGROUND

Lysine, of which the chemical name is 2,6-diaminopimelic acid, is an essential amino acid for animals and human body. It functions to promote growth, enhance immunity, and improve the function of the central nervous tissues. Lysine includes three optical isomers, namely L-form (levorotatory), D-form (dextrorotatory), and DL form (racemic), of which only the L-form is biologically utilizable, and the commonly referred lysine is L-lysine. Lysine is known as the first limited amino acid because cereal, the staple food of human, has a low content of lysine, while lack of lysine could lead to protein metabolic dysfunction and adverse effect on growth; also, lysine is susceptible to damage during processing. Lysine has a very important position in pharmaceutics, health, food, animal feed and cosmetic industries.

By far, there are three methods for industrial production of lysine, namely proteolysis method, chemical synthesis method, and microbial fermentation method. Among them, the microbial fermentation method has the advantages of low cost, high production intensity, high specificity, and low environmental pollution, and therefore becomes the most widely applied method in the industrial production of lysine. *Corynebacterium* and *Escherichia* bacteria are widely used in the industrial production of lysine. For example, commonly used *Escherichia* is *Escherichia coli*, commonly used *Corynebacterium* includes *Corynebacterium glutamicum* of the *Corynebacterium* genus, *Brevibacterium flavum* of the *Brevibacterium* genus, and some species of *Arthrobacterium* and some species of *Microbacterium.* Because of its physiological superiority, *Corynebacterium glutamicum* has become the most important strain for production in the industry.

*Corynebacterium glutamicum* has two pathways for lysine biosynthesis, i.e., the dehydrogenase pathway and the succinylase pathway. Lysine synthesis by *Corynebacterium glutamicum* through the dehydrogenase pathway involves six enzymes catalyzing reactions: aspartate kinase (AK, encoded by *lysC* gene), aspartate semialdehyde dehydrogenase (ASADH, encoded by *asd* gene), dihydrodipicolinate synthase (DHDPS, encoded by *dapA* gene), dihydrodipicolinate reductase (DHDPR, encoded by *dapB* gene), diaminopimelate dehydrogenase (DAPDH, encoded by *ddh* gene), and diaminopimelate decarboxylase (DAPDC, encoded by *lysA* gene). Lysine synthesis by *Corynebacterium glutamicum* through the succinylase pathway further involves four enzymes for the synthesis of meso-diaminopimelic acid: succinyl diaminopimelate aminotransferase (encoded by *dapD* gene), tetrahydropyridine dicarboxylate succinylase (encoded by *dapC* gene), succinyl diaminopimelate deacylase (encoded by *dapE* gene) and diaminopimelate epimerase (encoded by *dapF* gene). The first step of the L-aspartic acid biosynthetic pathway by *Corynebacterium glutamicum* is aspartate kinase catalyzing L-aspartate to produce aspartate phosphatase, which is also the rate-limiting step in lysine production. The activity of aspartate kinase is inhibited by feedback from lysine [1]. Therefore, how to remove the feedback inhibition of aspartate kinase by lysine in the lysine biosynthetic pathway is of high significance for the selective breeding of high lysine producing strains.

Reference Document 1 discloses mutants of aspartate kinase insensitive to lysine inhibition. By random mutagenesis of the encoding gene of aspartate kinase III (Aspartate kinase III, AKIII) isolated from *Escherichia coli*, two AK III mutants removing the feedback inhibition are screened and obtained, respectively substituting isoleucine for threonine at position 352 (T352I) and substituting isoleucine for methionine at position 318 (M318I). Although the two m AK III utants are capable of removing the feedback inhibition of lysine on aspartate kinase, they cannot result in high-yield lysine producing strains with significant increase of lysine yield due to their low lysine production efficiency.

Reference Document 2 discloses aspartate kinase mutants from *Corynebacterium glutamicum,* including 279T, A279V, S301F, T308I, S301Y, G345D, R320G, T311I, and S381F. These mutant AK III can remove the feedback inhibition of lysine on aspartate kinase, but vary dramatically in the efficiency of lysine production. T311I is a mutant reported to have the best performance in the application of lysine production and can significantly increase the lysine yield of the strains. To obtain a aspartate kinase mutant removing the feedback inhibition of lysine while achieving higher performance in lysine production is a problem urging to be solved by a person skilled in the art.

### Reference Documents:

Reference Document 1: EP1394257
Reference Document 2: EP1590463A2

### SUMMARY

### Problem to be solved

In view of the problems in the prior art, such as the problem that aspartate kinase mutants cannot produce lysine in a stable and highly efficient manner, the present disclosure provides a polypeptide with aspartate kinase activity, which is a mutant of the wild-type aspartate kinase. Compared with the wild-type aspartate kinase, the mutant polypeptide according to the present disclosure removes the feedback inhibition of lysine on aspartate kinase, has high aspartate kinase activity, and can be used for the stable and efficient production of lysine.

### Solution to the problem

(1) A polypeptide with aspartate kinase activity, wherein the polypeptide is selected from a group consisting of any one of (i) to (iv):
   (i) a mutant of the polypeptide having the sequence as shown in SEQ ID NO: 1, compared with the sequence as shown in SEQ ID NO: 1, the mutant comprising a mutation at least at one or more positions corresponding to positions 293, 294, and 307 of the sequence as shown in SEQ ID NO: 1;
   (ii) a polypeptide having at least 70%, at least 80%, or at least 90% sequence identity with the sequence as shown in (i) and not comprising the polypeptide having the sequence as shown in SEQ ID NO: 1;
   (iii) a polypeptide encoded by a polynucleotide, the polynucleotide being hybridized with the polynucleotide as shown in (a) or (b) under very high stringent conditions:
      (a) a polynucleotide encoding a polypeptide having the amino acid sequence as shown in (i);
      (b) a full-length complementary polynucleotide of (a);
   (iv) a fragment of the polypeptide as shown in (i), (ii), (iii), the fragment still having aspartate kinase activity.
(2) The polypeptide according to (1), wherein the polypeptide is a polypeptide comprising a mutation as shown in at least one group of (c) to (e) as follows:
   (c) the amino acid at a position corresponding to position 293 of the sequence as shown in SEQ ID NO: 1 being mutated from isoleucine (I) to serine (S), glycine (G), glutamic acid (E), proline (P), tryptophan (W), tyrosine (Y), histidine (H), methionine (M), glutamine (Q), cysteine (C), or arginine (R);
   (d) the amino acid at a position corresponding to position 294 of the sequence as shown in SEQ ID NO: 1 being mutated from aspartic acid (D) to tyrosine (Y), tryptophan (W), or phenylalanine (F);
   (e) the amino acid at a position corresponding to position 307 of the sequence as shown in SEQ ID NO: 1 being mutated from threonine (T) to tyrosine (Y), glycine (G), or phenylalanine (F).(3) The polypeptide according to (1) or (2), wherein the polypeptide comprises deletion or addition at least one amino acid residue at N-terminus or C-terminus of the polypeptide having the sequence as shown in (i).
(4) A recombinant polypeptide comprising the polypeptide according to any one of (1) to (3) and an exogenous polypeptide fused to the polypeptide; optionally, the exogenous polypeptide comprising a tag polypeptide; preferably, the exogenous polypeptide comprising a tag polypeptide and a spacer polypeptide linking the tag polypeptide to the polypeptide with aspartate kinase activity.
(5) An isolated polynucleotide, comprising a nucleotide sequence encoding the polypeptide according to any one of (1) to (3), or comprising a nucleotide sequence encoding the recombinant polypeptide according to (4).
(6) A nucleic acid construct, comprising the polynucleotide according to (5), the polynucleotide is operably linked to one or more regulatory sequence, the regulatory sequence directs production of polypeptides in an expression host.
(7) A recombinant expression vector, comprising the polynucleotide according to (5), or the nucleic acid construct according to (6).
(8) A recombinant host cell, comprising the polypeptide according to any one of (1) to (3), the recombinant polypeptide according to (4), the polynucleotide according to (5), the nucleic acid construct according to (6), or the recombinant expression vector according to (7).
(9) The recombinant host cell according to (8), wherein the host cell is from genus *Corynebacterium,* genus *Brevibacterium,* genus *Arthrobacterium,* genus *Microbacterium,* or genus *Escherichia*; preferably, the host cell is *Corynebacterium glutamicum* or *Escherichia coli.*
(10) Use of the polypeptide according to any one of (1) to (3), the recombinant polypeptide according to (4), the polynucleotide according to (5), the nucleic acid construct according to (6), the recombinant expression vector according to (7), or the recombinant host cell according to (8) or (9) in production of an amino acid;
   preferably, the amino acid being selected from lysine, threonine, isoleucine, and an amino acid derivative thereof, wherein the amino acid derivative includes at least one of pentanediamine, 5-aminovaleric acid, glutaric acid, and hydroxyisoleucine.
(11) A method for producing an amino acid, comprising steps of producing an amino acid using the polypeptide according to any one of (1) to (3), the recombinant polypeptide according to (4), the polynucleotide according to (5), the nucleic acid construct according to (6), the recombinant expression vector according to (7), or the recombinant host cell according to (8) or (9);
   optionally, the method further comprising a step of purifying or isolating the amino acid;
   preferably, the amino acid being selected from lysine, threonine, isoleucine, and an amino acid derivative thereof, wherein the amino acid derivative includes at least one of pentanediamine, 5-aminoglutaric acid, glutaric acid, and hydroxyisoleucine.

### Effects

In some embodiments, the present disclosure discovers a mutant of the wild-type aspartate kinase. The mutant removes the feedback inhibition of lysine on aspartate kinase, has high aspartate kinase activity, and can significantly increase lysine yield when applied in lysine production and realize stable and highly efficient lysine production. Further, the aspartate kinase mutant according to the present disclosure is highly efficient in the production of threonine, isoleucine, as well as amino acid derivatives such as pentanediamine, 5-aminovaleric acid, glutaric acid, and hydroxyisoleucine.

In some embodiments, the recombinant polypeptide, the isolated polynucleotide, the nucleic acid construct, and the recombinant expression vector according to the present disclosure each includes or expresses the above-mentioned polypeptide with aspartate kinase activity, and are applicable in the production of lysine and derivatives thereof.

In some embodiments, the recombinant host cell, the polypeptide with aspartate kinase activity removing the feedback inhibition of lysine, and the recombinant host cell according to the present application can accumulate excessive lysine, thus are suitable for industrial production of lysine and derivatives thereof.

In some embodiments, by using the polypeptide with aspartate kinase activity, the recombinant polypeptide, or the recombinant host cell, the method for producing an amino acid according to the present disclosure could realize stable and highly efficient production of amino acid. When applied to amino acid production, the method of the present disclosure could obtain high yield of lysine, threonine, isoleucine, and derivatives thereof.

### BRIEF DESCRIPTION OF THE DRAWINGS

- Fig. 1: shows a schematic diagram of pCas9gRNA-ccdB plasmid;
- Fig. 2: shows a schematic diagram of pRecT plasmid.

### DETAILED DESCRIPTION

### Definitions of Terms

When used in combination with the term "including" in the claims and/or specification, the word "a" or "an" may refer to "one", or refer to "one or more", "at least one", and "one or more than one".

As used in the claims and specification, the term "including", "having", "comprising", or "containing" is intended to be inclusive or open-ended, and does not exclude additional or unrecited elements or methods and steps.

Throughout the application document, the term "about" means that a value includes the error or standard deviation caused by the device or method used to measure the value.

It is applicable to the content disclosed herein that the term "or" is defined only as alternatives and "and/or", but the term "or" used herein refers to "and/or" unless otherwise expressly stated to be only alternatives or mutual exclusion between alternatives.

The selected/optional/preferred "numerical range", when used in the claims or the specification, includes not only the numerical endpoints at both ends of the range, but also all natural numbers covered between the above numerical endpoints with respect to these numerical endpoints.

As used herein, although other organic or inorganic catalysis can be used, the term "converting" refers to chemical conversion from one molecule to another molecule catalyzed mainly by one or more polypeptides (enzymes); alternatively, the term may refer to a ratio (in %) of the molar mass of an expected product to the molar mass of a limiting substrate.

As used herein, the term "aspartate kinase" and its abbreviation "AK" refer to a polypeptide (enzyme) that can catalyze the phosphorylation of aspartic acid into an aspartyl phosphate. As the first key enzyme for the lysine biosynthetic pathway, aspartate kinase controls the biosynthetic pathway for the amino acids of the Aspartate family.

As used herein, the terms "polypeptide", "peptide", and "protein" are used interchangeably herein and are an amino acid polymer of any length. This polymer may be linear or branched. It may include modified amino acids, and can be spaced by non-amino acids. This term also includes amino acid polymers that have already been modified (e.g., by formation of disulfide bonds, glycosylation, lipidization, acetylation, phosphorylation, or any other manipulation such as conjugation with a labeled component).

As used herein, the term "fragment" means a polypeptide or a catalytic or carbohydrate-binding module having one or more (e.g., several) amino acids deleted from the amino terminus and/or the carboxyl terminus of a mature peptide or a domain. According to the present disclosure, the fragment has aspartate kinase activity.

As used herein, the term "wild-type" refers to an object that can be found in nature. For example, a polypeptide or a polynucleotide sequence present in an organism that can be isolated from a natural source and has not been intentionally modified by human in the laboratory is naturally occurring. As used herein, "naturally occurring" and "wild-type" are synonymous.

The term "mutant" used herein refers to a polynucleotide or polypeptide including alternation(s) (i.e., substitution, insertion and/or deletion) at one or more (e.g., several) positions, as compared with the "wild-type" or "comparative" polynucleotide or polypeptide. Here, substitution refers to a substitution of a different nucleotide or amino acid for a nucleotide or amino acid that occupies one position; deletion refers to removal of a nucleotide or amino acid that occupies some position; and insertion refers to an addition of a nucleotide or amino acid after the nucleotide or amino acid adjacent to and immediately following the occupied position. Exemplarily, the "mutant" of the present disclosure is a polypeptide with increased aspartate kinase activity.

As used herein, the term "amino acid mutation" or "nucleotide mutation" includes "substituting, repeating, deleting or adding one or more amino acid or nucleotide". In the present disclosure, the term "mutation" refers to a change in a nucleotide sequence or amino acid sequence. In a particular embodiment, the term "mutation" means "substitution".

In some embodiments, "mutation" according to the present disclosure includes the substitution of amino acids at one or more positions corresponding to positions 293, 294, and 307 of the sequence as shown in SEQ ID NO: 1. Compared with the polypeptide having the sequence as shown in SEQ ID NO: 1, the mutant with the amino acid substitution at the above positions removes the feedback inhibition of lysine on enzyme activity, and improves the conversion rate from aspartic acid to aspartyl phosphate, thereby increasing lysine production.

In the present disclosure, "mutation" also includes adding, deleting, or substituting amino acids at one or more positions corresponding to the sequence as shown in SEQ ID NO: 1 without affecting aspartate kinase activity. It is known that changing a few amino acid residues in some region of a polypeptide, such as in an non-essential region, would not substantially alter the biological activity. For example, a sequence obtained by appropriate substitution, addition or deletion of some amino acids will not have affected biological activity^{[2]}. Exemplarily, the "mutation" of the present disclosure includes deletion or addition of at least one amino acid residue at a position corresponding to at least one of the C-terminus and the N-terminus of the polypeptide having the sequence as shown in SEQ ID NO: 1, and the mutant has aspartate kinase activity. In some embodiments, the "mutation" of the present disclosure includes deleting or adding, from the C-terminus or the N-terminus of the polypeptide having the sequence as shown in SEQ ID NO: 1, 1 to 20 amino acids, preferably 1 to 15 amino acids, more preferably 1 to 10 amino acids, more preferably 1 to 3 amino acids, and most preferably 1 amino acid, and the mutant has aspartate kinase activity.

In some embodiments, the "mutation" of the present disclosure may be selected from "conservative mutation". According to the present disclosure, the term "conservative mutation" refers to mutation that can maintain the normal function of proteins. A typical example of conservative mutation is conservative substitution.

As used herein, the term "conservative substitution" relates to substituting an amino acid residue for an amino acid residue having a similar side chain. The present field has defined amino acid residue families with similar side chains, including having basic side chains (e.g., lysine, arginine, and histidine), acid side chains (e.g., aspartic acid, glutamic acid), an uncharged polar side chain (e.g., glycine, asparagine, glutamine, serine, threonine, tyrosine, and cysteine), nonpolar side chains (e.g., alanine, valine, leucine, isoleucine, proline, phenylalanine, methionine, and tryptophan), β-branched side chains (e.g., threonine, valine, and isoleucine), and aromatic side chains (e.g., tyrosine, phenylalanine, tryptophan, and histidine).

As used herein, "conservative substitution" typically involves the substitution of one amino acid at one or more positions of a protein. Such substitution may be conservative. As exemplary substitutions deemed as conservative substitution, examples include substitutions of Ala to Ser or Thr; substitutions of Arg to Gln, His or Lys; substitutions of Asn to Glu, Gln, Lys, His or Asp; substitutions of Asp to Asn, Glu or Gln; substitutions of Cys to Ser or Ala; substitutions of Gln to Asn, Glu, Lys, His, Asp or Arg; substitutions of Glu to Gly, Asn, Gln, Lys or Asp; substitutions of Gly to Pro; substitutions of His to Asn, Lys, Gln, Arg or Tyr; substitutions of Ile to Leu, Met, Val or Phe; substitutions of Leu to Ile, Met, Val or Phe; substitutions of Lys to Asn, Glu, Gln, His or Arg; substitutions of Met to Ile, Leu, Val or Phe; substitutions of Phe to Trp, Tyr, Met, Ile or Leu; substitutions of Ser to Thr or Ala; substitutions of Thr to Ser or Ala; substitutions of Trp to Phe or Tyr; substitutions of Tyr to His, Phe or Trp; and substitutions of Val to Met, Ile or Leu. In addition, conservative mutation also includes naturally occurring mutations that arise from individual differences, and the differences among strains or species, from which the genes originate.

As used herein, the term "recombinant polypeptide" refers to a fusion expression of two or more polypeptides by a genetic engineering approach. In some embodiments, the recombinant polypeptide is recombinant polypeptide obtained by fusing a mutant of a polypeptide having the sequence as shown in SEQ ID NO: 1 with an exogenous polypeptide. In some embodiments, the exogenous polypeptide comprises a tag polypeptide. In some embodiments, the exogenous polypeptide comprises a tag polypeptide, and a spacer polypeptide linking the mutant to the tag polypeptide. Specifically, the spacer polypeptide may have less than 10 spacer amino acid residues.

Exemplarily, the tag polypeptide is a polypeptide having the tag sequence as shown in Table 1.

**Table 1 Tag Sequence**

| Tag | Number of Residue | Sequence |
|---|---|---|
| Poly-Arg | 5 or 6 (typically 5) | RRRRR |
| Poly-His | 2 to 10 (typically 6) | HHHHHH |
| FLAG | 8 | DYKDDDDK |
| Strep-tag II | 8 | WSHPQFEK |
| c-myc | 10 | EQKLISEEDL |
| HA | 9 | YPYDVPDYA |

In some embodiments, the recombinant polypeptide comprises, from N-terminus to C-terminus: a mutant of a polypeptide having the sequence as shown in SEQ ID NO: 1, a spacer polypeptide, and a tag polypeptide; wherein the tag polypeptide may be a polypeptide having the tag sequence as shown in His6, the spacer polypeptide has less than 10 spacer amino acid residues. For example, the amino acid sequence of the spacer polypeptide is "LE".

As used herein, the term "sequence identity" or "identity percentage" in the comparison of two nucleic acids or polypeptides means that, when compared and aligned at maximum correspondence by the sequence alignment algorithm or by the visual inspection measurement, they are the same or they have a specific percentage of identical sequence measured. That is, the identity of nucleotide or amino acid residue sequences could be defined to be such a ratio that is a ratio of the number of nucleotides or amino acid residues that are identical to the total number of nucleotide or amino acid residues in the two or more nucleotides or amino acid residues aligned to have the maximum number of identical nucleotides or amino acid residues, with gaps introduced as needed.

The method for measuring "sequence identity" or "identity percentage" involved in the present disclosure includes, but not limited to: Lesk, A.M. (Ed.). (1988). Computational Molecular Biology. New York: Oxford University Press.; Smith, D.W. (Ed.). (1993). Biocomputing Informatics and Genome Projects. New York: Academic Press.; Griffin, A.M. & Griffin, H.G. (Ed.). (1994). Computer Analysis of Sequence Data, Part I. New Jersey: Humana Press.; von Heinje G. (1987). Sequence Analysis in Molecular Biology. Academic Press.; Gribskov, M. & Devereux, J. (Ed.). (1991) Sequence Analysis Primer. New York: M Stockton Press.; and Carillo, H. & Lipman, D., SIAM J. Applied Math. 48:1073 (1988). A preferable method for measuring identity requires obtaining maximum correspondence of sequence. The method for measuring identity is compiled in a computer program accessible by the public. The preferable computer-program method for measuring identity between two sequences includes, but not limited to: GCG program package Devereux, J. et. al., 1984, BLASTP, BLASTN, and FASTA (Altschul, S. F. et. al., 1990). The public may acquire the BLASTX program from NCBI and other sources (Altschul, S. et. al. BLAST Manual. NCBI NLM NIH Bethesda, Md.20894; Altschul, S. et. al., 1990). The well-known Smith Waterman algorithm may also be used for measuring the identity.

In some embodiments, the polypeptide with aspartate kinase activity according to the present disclosure has at least 70%, 75%, 80%, 85%, 90%, 91%, 92%, 93%, 94%, 95%, 96%, 97%, 98% or 99% "sequence identity" or "identity percentage" of amino acid residues with the mutant of the polypeptide having the sequence as shown in SEQ ID NO: 1. In some other embodiments, the polynucleotide of the present disclosure for encoding the polypeptide with aspartate kinase activity has at least 70%, 75%, 80%, 85%, 90%, 91%, 92%, 93%, 94%, 95%, 96%, 97%, 98%or 99% "sequence identity" or "identity percentage" of nucleotide with the polynucleotide encoding the mutant of the polypeptide having the sequence as shown in SEQ ID NO: 1. Determination/calculation of the "sequence identity" or "identity percentage" may be based on any suitable region of the sequence, such as a region of a length of at least about 50 residues, at least about 100 residues, at least about 200 residues, at least about 400 residues, or at least about 500 residues. In some embodiments, the sequence is substantially identical at full length of any one or two biopolymers for comparison (e.g., nucleic acid or polypeptide).

As used herein, the term "polynucleotide" refers to a polymer composed of nucleotides. Polynucleotide can be in the form of a individual fragment or a component of a larger nucleotide sequence structure, and it is derived from a nucleotide sequence separated at least once in quantity or concentration and can be recognized, manipulated and restored from the sequence and its component nucleotide sequences by standard molecular biology methods (such as using a cloning vector). When a nucleotide sequence is represented by a DNA sequence (i.e., A, T, G, C), this also includes an RNA sequence (i.e., A, U, G, C), wherein "U" replaces "T". In other words, "polynucleotide" refers to a nucleotide polymer that has been removed from other nucleotides (an individual fragment or an entire fragment), or may be a constituent part or component of a larger nucleotide structure, such as an expression vector or a polycistronic sequence. The polynucleotide includes DNA, RNA and cDNA sequences.

As used herein, the term "isolated" refers to a substance in a form or environment that does not occur in nature. Non-limiting examples of isolated substances include (1) any substance that is not naturally occurring, (2) any substance including, but not limited to, any enzyme, mutant, nucleic acid, protein, peptide, or cofactor that is at least partially knocked out from one or more or all naturally occurring components associated with its nature; (3) any substance that is artificially modified relative to a naturally occurring substance; or (4) any substance that is modified by increasing the amount of the substance relative to other components associated with its nature (e.g., recombinant production in the host cell; multiple copies of the gene encoding the substance; and use of a promoter stronger than the promoter naturally associated with the gene encoding the substance). The isolated substance may be present in the fermentation broth sample. For example, host cells may be genetically modified to express the polypeptide of the present disclosure. The fermentation broth sample from the host cells will contain isolated polypeptides. The "recombinant polynucleotide" pertains to a "polynucleotide".

As used herein, the term "recombinant polynucleotide" refers to a polynucleotide having a sequence that is not linked together in nature. The recombinant polynucleotide may be included in a suitable vector that could be transformed to a suitable host cell. A host cell comprising a recombinant polynucleotide is referred to as a "recombinant host cell". Subsequently, the polynucleotide is expressed in the recombinant host cell to generate for example a "recombinant polypeptide".

As used herein, the term "expression" includes any step relating to the production of polypeptides, including, but not limited to: transcription, post-transcriptional modification, translation, post-translational modification, and secretion.

As used herein, the term "expression vector" refers to a linear or circular DNA molecule comprising a polynucleotide encoding a polypeptide, the polynucleotide effectively linked to a regulatory sequence for its expression.

As used herein, the term "recombinant expression vector" refers to a DNA structure for expressing for example a polynucleotide encoding the desired polypeptide. The recombinant expression vector may include, for example including i) a set of genetic elements regulating the gene expression, such as a promoter and a enhancer; ii) structures or coding sequences transcribed into mRNA and translated into proteins; and iii) transcriptional subunits that properly transcribes and translate the initiation and termination sequences. The recombinant expression vector may be constructed by any suitable method. The nature of the vector is not important; any vector may be used, including a plasmid, a virus, a phage, and a transposon. Possible vectors used in the present disclosure include, but not limited to, chromosomes, achromosomes, and synthesized DNA fragments, such as bacterial plasmid, phage DNAs, yeast plasmids, and vectors derived from a combination of plasmids and phage DNAs, virus DNAs from for example cowpox, adenovirus, fowl pox, baculovirus, SV40, and pseudorabies virus.

As used herein, the term "recombinant gene" refers to genes that are not naturally occurring. A recombinant gene includes a protein-coding sequence operably linked to an expression regulatory sequence. Examples include, but not limited to: exogenous genes introducing microbes, endogenous protein-coding sequences operably linked to a heterelogous promoter, and genes having a modified protein-coding sequence. Recombinant genes are stored in genomes of microbiomes, plasmids in microbiomes, or phages in microbiomes.

As used herein, the term "operably linked" refers to a construction in which a regulatory sequence is placed at a proper position with respect to the coding sequence of the polynucleotide such that the regulatory sequence guides the expression of the coding sequence. Exemplarily, the regulatory sequence may be selected from a promoter and/or enhancer-coding sequence.

As used herein, the term "nucleic acid construct" includes a polynucleotide effectively linked to a suitable regulatory sequence and encoding a polypeptide or a domain or a module, the regulatory sequence being necessary for the polynucleotide expression of the selected cells or strands. In the present disclosure, the transcriptional regulator comprises a promoter. Further, it may comprise an element such as an enhancer, a silencer, and an insulator.

The term "host cell" used herein refers to any type of cells that may be easily transformed, transfected, or transduced with a mutant polypeptide of the present disclosure, a polynucleotide encoding the mutant polypeptide or a recombinant expression vector. The term "recombinant host cell" covers a host cell that is different from the parent cells after introducing the polynucleotide encoding a mutant polypeptide or a recombinant expression vector. The host cell of the present disclosure may be a prokaryotic cell or a eukaryotic cell, as long as it is a cell into which a polynucleotide encoding the polypeptide and recombinant polypeptide with aspartate kinase activity of the present disclosure can be introduced. In an embodiment, the host cell refers to a prokaryotic cell. More specifically, the host cell is derived from microbiomes suitable for production of an amino acid by fermentation, such as genus *Corynebacterium,* genus *Brevibacterium,* genus *Microbacterium,* or genus *Escherichia.* Preferably, the host cell is derived from *Corynebacterium glutamicum* of genus *Corynebacterium,* or is derived from *Escherichia coli* of genus *Escherichia.*

The terms "transformation, transfection, transduction" used herein has the meaning generally understood by a person skilled in the art, *i.e*., a process of introducing an exogenous DNA into a host. Methods for transformation, transfection, and transduction include any method for introducing a nucleic acid into a cell. These methods include, but are not limited to, electroporation, calcium phosphate (CaPO₄) precipitation, calcium chloride (CaCh) precipitation, microinjection, a polyethylene glycol (PEG) method, a DEAE-dextran method, a cationic liposome method, and a lithium acetate-DMSO method.

In the present disclosure, the host cells may be cultured by conventional methods in the art, including, but not limited to, well-plate culture, shaking culture, batch culture, continuous culture, fed batch culture, etc. Also, various culturing conditions such as temperature, time, and pH value of the medium may be properly adjusted according to actual situations.

As used herein, the term "high-stringent conditions" means that following the standard DNA blotting procedures, a probe of at least 100 nucleotides in length pre-hybridizes or hybridizes for 12 to 24 hours at 42°C in 5X SSPE (saline sodium phosphate EDTA), 0.3% SDS, 200 µg/ml of cleaved and denatured salmon sperm DNA, and 50% formamide. Finally, the vector material is washed three times at 65°C with 2X SSC and 0.2% SDS, each for 15 min.

As used herein, the term "very high-stringent conditions" means that following the standard DNA blotting procedures, a probe of at least 100 nucleotides in length pre-hybridizes or hybridizes for 12 to 24 hours at 42°C in 5X SSPE (saline sodium phosphate EDTA), 0.3% SDS, 200 µg/ml of cleaved and denatured salmon sperm DNA, and 50% formamide. Finally, the vector material is washed three times at 70°C with 2X SSC and 0.2% SDS, each for 15 min.

Unless otherwise defined or clearly specified in the background, all technical and scientific terms used herein have the same meanings as typically understood by one of ordinary skill in the art to which the present disclosure belongs.

### Mutants of Aspartate Kinase

In some embodiments, the present disclosure constructed a library of *LysC* gene mutants encoding aspartate kinase in *Corynebacterium glutamicum* by site-directed mutagenesis. From the library, mutants that could remove the feedback inhibition of lysine and thereby increase aspartate kinase activity were screened.

In some embodiments of the present disclosure, the mutation positions of mutants removing the lysine feedback inhibition include amino acids substituted at one or more positions corresponding to positions 293, 294 and 307 of the sequence as shown in SEQ ID NO: 1.

Exemplarily, the mutation at position 293 includes an amino acid mutated from isoleucine (I) to serine (S), glycine (G), glutamic acid (E), proline (P), tryptophan (W), tyrosine (Y), histidine (H), methionine (M), glutamine (Q), cysteine (C), or arginine (R).

The mutation at position 294 includes an amino acid mutated from aspartic acid (D) to tyrosine (Y), tryptophan (W), phenylalanine (F).

The mutation at position 307 includes an amino acid mutated from threonine (T) to tyrosine (Y), glycine (G), phenylalanine (F).

In some embodiments, the present disclosure provides a polypeptide with aspartate kinase activity, having at least 70%, 75%, 80%, 85%, 90%, 91%, 92%, 93%, 94%, 95%, 96%, 97%, 98%, 99%, 100% sequence identity to the polypeptide having the sequence as shown in SEQ ID NO: 1, and having an amino acid sequence that is not SEQ ID NO: 1.

In some embodiments, the present disclosure provides a polypeptide with aspartate kinase activity, comprising addition or deletion of amino acids from at least one of the N-terminus and the C-terminus of the mutant of aspartate kinase.

In some particular embodiments, the mutant of aspartate kinase has deletion or addition of 1 to 20 amino acids, preferably 1 to 15 amino acids, more preferably 1 to 10 amino acids, more preferably 1 to 3 amino acids, and most preferably 1 amino acid, from at least one of the N-terminus and the C-terminus, and has aspartate kinase activity.

In some embodiments, the present disclosure provides a polynucleotide encoding the mutant of aspartate kinase, the polynucleotide encoding a mutant of a polypeptide having the sequence as shown in SEQ ID NO: 1. Specifically, the polynucleotide is a *LysC* gene mutant of high lysine production that is screened from the library of *LysC* gene mutants. The nucleotide sequence of *LysC* gene is as shown in SEQ ID NO: 2.

In some embodiments, the corresponding nucleotide sequence of the polynucleotide encoding the amino acid of position 293 is selected from any one of the following groups: TCC; GGC; GAA; CCA; TGG; TAC; CAC; ATG; CAA; TGC; CGC. Preferably, the corresponding nucleotide sequence of the polynucleotide encoding the amino acid of position 293 is selected from any one of the following groups: TCC; GGC; GAA; CCA; TGG; TAC; CAC; ATG; CAA.

In some embodiments, the corresponding nucleotide sequence of the polynucleotide encoding the amino acid of position 294 is selected from any one of the following groups: TAC; TGG; TTC. Preferably, the corresponding nucleotide sequence of the polynucleotide encoding the amino acid of position 294 is selected from any one of the following groups: TGG; TTC.

In some embodiments, the corresponding nucleotide sequence of the polynucleotide encoding the amino acid of position 307 is selected from any one of the following groups: TAC; GGC; TTC. Preferably, the corresponding nucleotide sequence of the polynucleotide encoding the amino acid of position 307 is selected from any one of the following groups: TAC; GGC.

### Construction of the library of LysC gene mutants

With the pCas9 plasmid ^{[3]} as a template and *cas*9-1/*cas*9-2 as amplification primers, an amplified fragment 1 comprising Cas9 was amplified, wherein operating element lacO mutation and RBS mutation were introduced into the primers *cas*9-1/*cas*9-2.

With pnCas9(D10A)-AID-gRNA-*ccdB*^{TS [4]} as a template and gRNA-1/gRNA-2 as amplification primers, an amplified fragment 2 comprising a gRNA-*ccdB* expression cassette and a temperature sensitive replication origin was amplified.

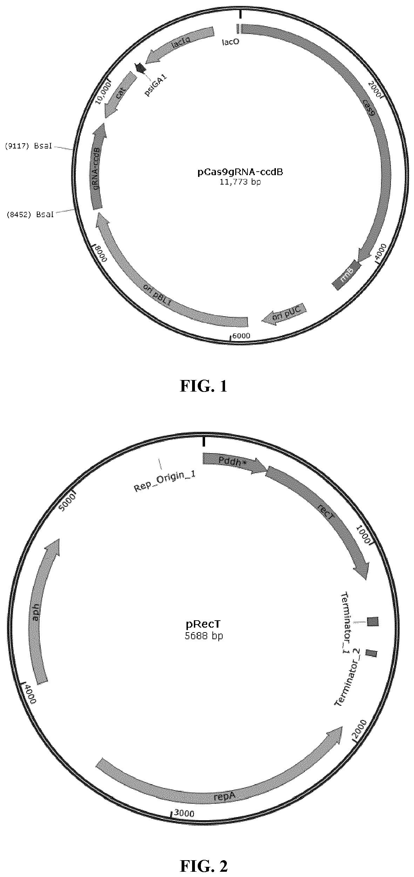

The amplified fragment 1 and the amplified fragment 2 were recombinantly linked, obtaining the pCas9gRNA-*ccdB* plasmid. The obtained pCas9gRNA-*ccdB* plasmid had a plasmid vector map as shown in Fig. 1 and a sequence as shown in SEQ ID NO: 3.

With the *Corynebacterium glutamicum* ATCC13032 genome as a template and P*_{ddh}*-1/P*_{ddh}*-2 as primers, a promoter mutant comprising the *ddh* gene was amplified as an amplified fragment 3.

With the Escherichia coli MG1655 genome as a template and *recT*-1/*recT-*2 as primers, the *recT* gene was amplified as an amplified fragment 4.

With the pEC-XK99E plasmid as a template and *rrnB*-1/*rrnB*-2 as primers, the *rrnB* terminator was amplified as an amplified fragment 5.

With the pEC-XK99E plasmid as a template and PEC-1/ PEC-2 as primers, a fragment of the plasmid skeleton from which the *per* gene was knocked out was amplified as an amplified fragment 6.

The amplified fragment 3, the amplified fragment 4, the amplified fragment 5, and the amplified fragment 6 were recombinantly linked, obtaining the pRecT plasmid expressing RecT. The pRecT plasmid had a plasmid vector map as shown in Fig. 2 and a sequence as shown in SEQ ID NO: 5.

The pCas9gRNA plasmids respectively targeting positions 293/294 and 307 of *lysC* gene were constructed by Goldengate Cloning method, wherein the target DNA-binding region of gRNA-1 was TGCAGAAATCAACATTGACA, the target DNA-binding region of gRNA-2 was GCAGGTGAAGGTGATGTCGG. Specifically, *lysC-*F1/*lysC-*R1, *lysC-*F2/*lysC-*R2 were denatured and annealed, and were then subjected to Goldengate Cloning together with the pCas9gRNA-*ccdB* plasmid, respectively, obtaining pCas9gRNA-*lysC*1 plasmid and pCas9gRNA-*lysC*2 plasmid.

The pRecT plasmid was electrotransformed into *Corynebacterium glutamicum* ATCC13032, obtaining the ATCC13032 (pRecT) strain. The single-stranded DNAs of 293-1 to 293-9, 294-1 to 294-9 and the single-stranded DNAs of 307-1 to 307-9 were designed respectively, as recombination templates for mutant construction. The pCas9gRNA-*lysC*1 plasmid and the single-stranded DNAs of 293-1 to 293-9, 294-1 to 294-9, and the pCas9gRNA-*lys*C2 plasmid and the single-stranded DNAs of 307-1 to 307-9 were respectively introduced into ATCC13032 (pRecT) competent cells. The competent cells transfected with the plasmids and single-stranded DNAs were coated on a plate, respectively. Monoclonal strains comprising amino acid mutations at positions 293, 294, 307 were screened, obtaining the library of *LysC* gene mutants.

In some embodiments, the competent cells transfected with the target DNA were added into 1 mL TSB liquid medium pre-heated at 46 °C, subjected to heat shock at 46 °C for 6 min, incubated at 30°C for 3 h, coated on TSB plates supplemented with 5 µg/mL chloramphenicol and 0.05 mM IPTG and incubated for 2 days, to grow hundreds of clones which were screened to obtain a library of mutants at amino acid positions 293, 294, 307.

The ingredients (g/L) of the TSB liquid medium were as follows: glucose, 5 g/L; yeast powder, 5 g/L; soy peptone, 9 g/L; urea, 3 g/L; succinic acid, 0.5 g/L; K₂HPO₄·3H₂O, 1 g/L; MgSO₄·7H₂O, 0.1 g/L; biotin, 0.01 mg/L; vitamin B1, 0.1 mg/L; MOPS, 20 g/L. The solid medium was supplemented with 15 g/L agar powder.

The *Corynebacterium glutamicum* is specifically *Corynebacterium glutamicum* ATCC 13032 (*Corynebacterium glutamicum ATCC 13032,* Gene ID: 2830649).

### Production process of amino acids

(1) Monoclonal strains with high lysine production were screened in the library of *LysC* gene mutants with amino acid mutations at positions 293, 294, 307 by a method that reproducibly yielded the screening results as recombinant host cells for amino acid production.
(2) The recombinant host cells were subjected to fermentation culture. Amino acids were collected from the recombinant host cells or the culture broth of the recombinant host cells, thereby completing the production of amino acids.

During the above production process of amino acids, the mutant *LysC* genes comprised by the recombinant host cells could encode the aspartate kinase removing lysine inhibition. The recombinant host cells could accumulate a huge amount of lysine, realizing stable and highly effective production of lysine, threonine, isoleucine, and derivatives thereof.

For the produced amino acids, the amino acids are selected from lysine, threonine, isoleucine, and an amino acid derivative thereof, wherein the amino acid derivative thereof includes at least one of pentanediamine, 5-aminovaleric acid, glutaric acid, and hydroxyisoleucine.

For the recombinant host cells, a recombinant host cell may also comprise other enzymes associated with synthesis of amino acids. Exemplarily, enzymes associated with synthesis of amino acids include a combination of one or two of: aspartate kinase, aspartate semialdehyde dehydrogenase, aspartate ammonia lyase, dihydrodipicolinate synthase, dihydropyridinic acid reductase, succinyl diaminopimelate aminotransferase, tetrahydrodipicolinate succinylase, succinyl diaminopimelate deacylase, diaminopimelic acid epimerase, diaminopimelic acid deacylase, glyceraldehyde-3-phosphate dehydrogenase, lysine transport protein, transketolase, diaminopimelate dehydrogenase, and pyruvate carboxylase.

In some embodiments, activity or expression of the enzyme associated with synthesis of amino acids may be enhanced by gene engineering, for example, by introducing strong promoters or by means of expression by free plasmids to increase the expression intensity of the genes encoding the enzymes associated with the synthesis of amino acids, or by chromosomal integration to integrate genes encoding enzymes associated with the synthesis of amino acids with higher enzymatic activity from other species originates.

In some embodiments, the host cell was *Corynebacterium glutamicum.* In some other embodiments, the host cell was *Escherichia coli. Corynebacterium glutamicum* and *Escherichia coli* are both important strains for lysine production. By expressing the aspartate kinase mutant removing the feedback inhibition of lysine in *Corynebacterium glutamicum* and *Escherichia coli,* it is possible to realize highly efficient production of lysine and derivatives thereof.

In some embodiments, the conditions for screening recombinant host cells were as follows: the strains were inoculated with toothpicks into a 96-well plate containing 200 µl fermentation medium in each well and cultured at 30°C for 24 h. The rotating speed of the plate shaker was 800 rpm.

In some embodiments, the conditions for fermentation culture were as follows: the recombinant host cells were inoculated into a TSB liquid medium and cultured for 6-8 h. The cultures were inoculated as seeds into a 24-well plate containing 600 µl fermentation medium in each well with the initial OD₆₀₀ controlled at about 0.1, and cultured at 30°C for 17 h. The rotating speed of the plate shaker was 800 rpm. Three samples were set for each strain. After the fermentation, OD₆₀₀ and the lysine yield were measured.

The ingredients of the fermentation medium were as follows: glucose, 80 g/L; yeast powder, 1 g/L; soy peptone, 1 g/L; NaCl, 1 g/L; ammonium sulfate, 1 g/L; urea, 8 g/L; K₂HPO₄·3H₂O, 1 g/L; MgSO₄·7H₂O, 0.45 g/L; FeSO₄·7H₂O, 0.05 g/L; biotin, 0.4 mg/L; vitamin B1, 0.1 mg/L; MOPS, 40 g/L; and initial pH7.2.

In some embodiments, for recovering amino acids from the recombinant host cells or the culture broth of the recombinant cells, a common method in this field may be used, including but not limited to: filtration, anion exchange chromatography, crystallization, and HPLC.

The enzymes catalyzing some or all of the reactions described in the present disclosure may be expressed in non-natural, engineered heterologous organisms. Specifically, genes encoding the enzyme for the synthesis pathway may be isolated, inserted into expression vectors of organisms used for transformation production, and incorporated into a genome, and directly express the enzyme. In this field, the method for manipulating microorganisms are known, such as explained in Current Protocols in Molecular Biology (Online ISBN: 9780471142720, John Wiley and Sons, Inc.), Microbial Metabolic Engineering: Methods and Protocols (Qiong Cheng Ed., Springer), and Systems Metabolic Engineering: Methods and Protocols (Hal S. Alper Ed., Springer).

### Examples

Additional objectives, features, and advantages of the present disclosure are apparent from the subsequent description in detail. However, it is to be appreciated that the detailed description and specific examples (although indicating specific embodiments of the present disclosure) are provided merely for illustrative purpose. Based on the detailed description, various changes and modifications within the spirits and the scopes of the present disclosure will become apparent to a person skilled in the art.

The experimental techniques and experimental methods used in the examples, unless otherwise specified, are all conventional techniques and methods, for example, experimental methods for which no specific conditions are indicated in the following examples are generally performed according to conventional conditions such as those described by Sambrook et al., Molecular Cloning: A Laboratory Manual (New York: Cold Spring Harbor Laboratory Press, 1989), or those recommended by manufacturers. The materials, reagents, etc. used in the examples, unless otherwise specified, are all commercially available.

### Example 1. Construction of a library of Corynebacterium glutamicum lysC gene mutants

Based on the sequence and the structural characteristics of *Corynebacterium glutamicum* LysC and the inventor's understanding of its functions, it was presumed that mutations of amino acids at positions 293, 294, 307 could remove lysine feedback inhibition of LysC, and increase L-lysine yield of *Corynebacterium glutamicum.*

For rapid site-directed mutation of *Corynebacterium glutamicum,* the present disclosure firstly constructed an editing system for single-strand recombination-based CRISPR/Cas9 genome. With the pCas9 plasmid ^{[3]} as a template and *cas*9-1/*cas*9-2 as primers, the operating element lacO mutation (TGTGTGGAATTGTGAGCGGATAACAATTTCACACA mutated to TGTGTGGAATTGTGAGCGCTCACAATTTCACACA) and RBS mutation (AAAGGAGTTGAGA mutated to AAAGGCACCCGAT) were introduced into the primers to amplify the fragments comprising *cas9*; then, with pnCas9(D10A)-AID-gRNA-*ccdB*^{TS[4]} plasmid as a template and gRNA-1/gRNA-2 as primers, a fragment of the plasmid skeleton comprising a gRNA - *ccdB* expression cassette and a temperature sensitive replication origin was amplified. The above 2 fragments were ligated via Vazyme's One Step Cloning Kit to obtain a highly efficiently constructed pCas9gRNA-*ccdB* plasmid. The plasmid had a plasmid vector map as shown in Fig. 1 and a sequence as shown in SEQ ID NO: 3. With the *Corynebacterium glutamicum* ATCC13032 genome as a template and P*_{ddh}-*1/ P*_{ddh}*-2 as primers, the *ddh* gene promoter mutant (ATGCATCTC mutated to ACAAAAGGT) was amplified, of which the sequence is as shown in SEQ ID NO: 4; with the *Escherichia coli* MG1655 genome as a template, the *recT* gene was amplified; with the pEC-XK99E plasmid as a template and *rrnB*-1/*rrnB*-2 as primers, the *rrnB* terminator fragment was amplified; with the pEC-XK99E plasmid as a template and PEC-1/ PEC-2 as primers, a fragment of the plasmid skeleton from which the *per* gene was knocked out was amplified. The above 4 fragments were ligated via Vazyme's One Step Cloning Kit to obtain a pRecT plasmid that expressing RecT. The plasmid had a plasmid vector map as shown in Fig. 2 and a sequence as shown in SEQ ID NO: 5.

Two pCas9gRNA plasmids respectively targeting positions 293/294 and 307 of the *lysC* gene were constructed by the Goldengate Cloning method^{[4]} reported in the literature. The target DNA-binding region of gRNA1 was TGCAGAAATCAACATTGACA; the target DNA-binding region of gRNA2 was GCAGGTGAAGGTGATGTCGG. The *lysC-*F1/*lysC*-R1, *lysC*-F2/*lysC-*R2 primers were denatured and annealed, respectively, and then were subjected to Goldengate Cloning together with the pCas9gRNA-*ccdB* plasmid, obtaining pCas9gRNA-*lysC*1 and pCas9gRNA-lysC2 plasmid, respectively.

On the basis of the single-stranded recombinant CRISPR/Cas9 genome editing system, a library of *lysC* gene mutants at amino acid positions 293, 294 and 307 were constructed. The pRecT plasmid was electrotransformed into *Corynebacterium glutamicum* ATCC13032, obtaining the ATCC13032 (pRecT) strain. Competent cells ^{[5]} were prepared from ATCC13032 (pRecT) strain using the method reported in the literature. In order to perform 19 mutations other than the wild-type mutations at amino acid positions 293, 294 and 307 of the *lysC* gene, the single-stranded DNAs of 293-1 to 293-9, 294-1 to 294-9 and that of 307-1 to 307-9 were designed as the recombination template for mutant construction. The competent cells of ATCC13032 (pRecT) were electrotransformed respectively with 1-2 µg pCas9gRNA-*lysC*1 plasmid and 10 µg single-stranded DNAs of equimolar concentration of the theoretical 19 mutants of 293-1 to 293-9, and with 1-2 µg pCas9gRNA-*lysC*1 plasmid and 10 µg single-stranded DNAs of equimolar concentration of the theoretical 19 mutants of 294-1 to 294-9, and with 1-2 µg pCas9gRNA-*lysC*2 plasmid and 10 µg single-stranded DNAs of equimolar concentration of the theoretical 19 mutants of 307-1 to 307-9, and then added into 1 mL TSB culture medium pre-heated at 46°C, subj ected to heat shock at 46°C for 6 min, incubated at 30 °C for 3 h, coated on TSB plates supplemented with 5 µg/mL chloramphenicol and 0.05 mM IPTG and incubated for 2 days, to grow hundreds of clones, obtaining a library of mutants at amino acid positions 293, 294 and 307, respectively. The ingredients (g/L) of the TSB liquid medium were as follows: glucose, 5 g/L; yeast powder, 5 g/L; soy peptone, 9 g/L; urea, 3 g/L; succinic acid, 0.5 g/L; K₂HPO₄·3H₂O, 1 g/L; MgSO₄·7H₂O, 0.1 g/L; biotin, 0.01 mg/L; vitamin B1, 0.1 mg/L; MOPS, 20 g/L. The solid medium was supplemented with 15 g/L FO agar powder. The sequences of the primers used above are as shown in Table 2.

**Table 2**

| Primer | Nucleotide Sequence | SEQ ID NO. |
|---|---|---|
| *cas9-1* | GCGCTCACAATTTCACACAGGAAACAGAATTAATTAAGC TTAAAGGCACCCGATATGGATAAGAAATACTCAATAGGC | SEQ ID NO: 6 |
| *cas9-2* | TCAGTCACCTCCTAGCTGACTCAAATC | SEQ ID NO: 7 |
| gRNA-1 | GTCAGCTAGGAGGTGACTGAAGCTTGGCTGTTTTGGCGG ATG | SEQ ID NO: 8 |
| gRNA-2 | CTGTGTGAAATTGTGAGCGCTCACAATTCC | SEQ ID NO: 9 |
| P*_{ddh}*-1 | CTGTGCGGTATTTCACACCGGTGCGTGGCGAGTTTTACA AAG | SEQ ID NO: 10 |
| P*_{ddh}*-2 | GTTCTTGTAATCCTCCAAAATTGTGGTGGCACTGTCCTGG TCGAGCTTACCACCTTTTGTACTTAGATGATGATTCAGGG AC | SEQ ID NO: 11 |
| *recT-1* | TTTTGGAGGATTACAAGAACATGACTAAGCAACCACCAA TCGC | SEQ ID NO: 12 |
| *recT-2* | TTCCTCTGAATTATCGATTACACTG | SEQ ID NO: 13 |
| *rrnB-1* | TAATCGATAATTCAGAGGAATAACTGTTTTGGCGGATGAG AGAAG | SEQ ID NO: 14 |
| *rrnB-2* | GGAGACCTTTTTGGAGCTAGTCG | SEQ ID NO: 15 |
| PEC-1 | CTAGCTCCAAAAAGGTCTCCAGG | SEQ ID NO: 16 |
| PEC-2 | CGGTGTGAAATACCGCACAGATG | SEQ ID NO: 17 |
| *lysC-F 1* | TTCATGCAGAAATCAACATTGACA | SEQ ID NO: 18 |
| *lysC-R 1* | AAACTGTCAATGTTGATTTCTGCA | SEQ ID NO: 19 |
| *lysC-F2* | TTCAGCAGGTGAAGGTGATGTCGG | SEQ ID NO: 20 |
| *lysC-R2* | AAACCCGACATCACCTTCACCTGC | SEQ ID NO: 21 |
| 293-1 | GGTGGTGCCGTCTTCTACAGAAGAGACGTTCTGCAGAAC CATGTCGARGTTGATTTCTGCATCAGCCAACGCACGGAA AACCTTCGCAGC | SEQ ID NO: 22 |
| 293-2 | GGTGGTGCCGTCTTCTACAGAAGAGACGTTCTGCAGAAC CATGTCCWTGTTGATTTCTGCATCAGCCAACGCACGGAA | SEQ ID NO: 23 |
| | AACCTTCGCAGC | |
| 293-3 | GGTGGTGCCGTCTTCTACAGAAGAGACGTTCTGCAGAAC CATGTCGGHGTTGATTTCTGCATCAGCCAACGCACGGAA AACCTTCGCAGC | SEQ ID NO: 24 |
| 293-4 | GGTGGTGCCGTCTTCTACAGAAGAGACGTTCTGCAGAAC CATGTCGTDGTTGATTTCTGCATCAGCCAACGCACGGAA AACCTTCGCAGC | SEQ ID NO: 25 |
| 293-5 | GGTGGTGCCGTCTTCTACAGAAGAGACGTTCTGCAGAAC CATGTCTKGGTTGATTTCTGCATCAGCCAACGCACGGAA AACCTTCGCAGC | SEQ ID NO: 26 |
| 293-6 | GGTGGTGCCGTCTTCTACAGAAGAGACGTTCTGCAGAAC CATGTCWTCGTTGATTTCTGCATCAGCCAACGCACGGAA AACCTTCGCAGC | SEQ ID NO: 27 |
| 293-7 | GGTGGTGCCGTCTTCTACAGAAGAGACGTTCTGCAGAAC CATGTCGCVGTTGATTTCTGCATCAGCCAACGCACGGAA AACCTTCGCAGC | SEQ ID NO: 28 |
| 293-8 | GGTGGTGCCGTCTTCTACAGAAGAGACGTTCTGCAGAAC CATGTCGACGTTGATTTCTGCATCAGCCAACGCACGGAA AACCTTCGCAGC | SEQ ID NO: 29 |
| 293-9 | GGTGGTGCCGTCTTCTACAGAAGAGACGTTCTGCAGAAC CATGTCCCAGTTGATTTCTGCATCAGCCAACGCACGGAA AACCTTCGCAGC | SEQ ID NO: 30 |
| 294-1 | GTCGGTGGTGCCGTCTTCTACAGAAGAGACGTTCTGCAG AACCATGADAATGTTGATTTCTGCATCAGCCAACGCACG GAAAACCTTCGC | SEQ ID NO: 31 |
| 294-2 | GTCGGTGGTGCCGTCTTCTACAGAAGAGACGTTCTGCAG AACCATCWTAATGTTGATTTCTGCATCAGCCAACGCACG GAAAACCTTCGC | SEQ ID NO: 32 |
| 294-3 | GTCGGTGGTGCCGTCTTCTACAGAAGAGACGTTCTGCAG AACCATGGHAATGTTGATTTCTGCATCAGCCAACGCACG GAAAACCTTCGC | SEQ ID NO: 33 |
| 294-4 | GTCGGTGGTGCCGTCTTCTACAGAAGAGACGTTCTGCAG AACCATGTDAATGTTGATTTCTGCATCAGCCAACGCACG GAAAACCTTCGC | SEQ ID NO: 34 |
| 294-5 | GTCGGTGGTGCCGTCTTCTACAGAAGAGACGTTCTGCAG AACCATTKGAATGTTGATTTCTGCATCAGCCAACGCACG GAAAACCTTCGC | SEQ ID NO: 35 |
| 294-6 | GTCGGTGGTGCCGTCTTCTACAGAAGAGACGTTCTGCAG AACCATTTCAATGTTGATTTCTGCATCAGCCAACGCACGG AAAACCTTCGC | SEQ ID NO: 36 |
| 294-7 | GTCGGTGGTGCCGTCTTCTACAGAAGAGACGTTCTGCAG AACCATGCVAATGTTGATTTCTGCATCAGCCAACGCACG GAAAACCTTCGC | SEQ ID NO: 37 |
| 294-8 | GTCGGTGGTGCCGTCTTCTACAGAAGAGACGTTCTGCAG AACCATGACAATGTTGATTTCTGCATCAGCCAACGCACG GAAAACCTTCGC | SEQ ID NO: 38 |
| 294-9 | GTCGGTGGTGCCGTCTTCTACAGAAGAGACGTTCTGCAG AACCATCCAAATGTTGATTTCTGCATCAGCCAACGCACG GAAAACCTTCGC | SEQ ID NO: 39 |
| 307-1 | CGCGCGGCGGCCGTCGGAACGAGGGCAGGTGAAGGTGA TGTCGGTGADGCCGTCTTCTACAGAAGAGACGTTCTGCA GAACCATGTCAAT | SEQ ID NO: 40 |
| 307-2 | CGCGCGGCGGCCGTCGGAACGAGGGCAGGTGAAGGTGA TGTCGGTCWTGCCGTCTTCTACAGAAGAGACGTTCTGCA GAACCATGTCAAT | SEQ ID NO: 41 |
| 307-3 | CGCGCGGCGGCCGTCGGAACGAGGGCAGGTGAAGGTGA TGTCGGTAGMGCCGTCTTCTACAGAAGAGACGTTCTGCA GAACCATGTCAAT | SEQ ID NO: 42 |
| 307-4 | CGCGCGGCGGCCGTCGGAACGAGGGCAGGTGAAGGTGA TGTCGGTGTDGCCGTCTTCTACAGAAGAGACGTTCTGCA GAACCATGTCAAT | SEQ ID NO: 43 |
| 307-5 | CGCGCGGCGGCCGTCGGAACGAGGGCAGGTGAAGGTGA TGTCGGTTKGGCCGTCTTCTACAGAAGAGACGTTCTGCA GAACCATGTCAAT | SEQ ID NO: 44 |
| 307-6 | CGCGCGGCGGCCGTCGGAACGAGGGCAGGTGAAGGTGA TGTCGGTWTCGCCGTCTTCTACAGAAGAGACGTTCTGCA GAACCATGTCAAT | SEQ ID NO: 45 |
| 307-7 | CGCGCGGCGGCCGTCGGAACGAGGGCAGGTGAAGGTGA | SEQ ID NO: 46 |
| | TGTCGGTGCVGCCGTCTTCTACAGAAGAGACGTTCTGCA GAACCATGTCAAT | |
| 307-8 | CGCGCGGCGGCCGTCGGAACGAGGGCAGGTGAAGGTGA TGTCGGTGACGCCGTCTTCTACAGAAGAGACGTTCTGCA GAACCATGTCAAT | SEQ ID NO: 47 |
| 307-9 | CGCGCGGCGGCCGTCGGAACGAGGGCAGGTGAAGGTGA TGTCGGTCCAGCCGTCTTCTACAGAAGAGACGTTCTGCA GAACCATGTCAAT | SEQ ID NO: 48 |
| *lysC-*C1 | CGGACGTTGACGGTGTGTATAC | SEQ ID NO: 49 |
| *lysC-*C2 | GCACGGAAATACGAATCTCAGAG | SEQ ID NO: 50 |

### Example 2. Screening and sequencing of the library of Corynebacterium glutamicum lysC gene mutants

In order to screen the library of *Corynebacterium glutamicum lysC* gene mutants, 60 clones were selected for each of positions 293, 294 and 307 from the library of mutants and subjected to fermentation, so as to screen the mutants of high L-lysine yield. The ingredients of the fermentation medium were as follows: glucose, 80 g/L; yeast powder, 1 g/L; soy peptone, 1 g/L; NaCl, 1 g/L; ammonium sulfate, 1 g/L; urea, 8 g/L; K₂HPO₄·3H₂O, 1 g/L; MgSO₄·7H₂O, 0.45 g/L; FeSO₄·7H₂O, 0.05 g/L; biotin, 0.4 mg/L; vitamin B1, 0.1 mg/L; MOPS, 40 g/L; and initial pH7.2. The strains were inoculated with toothpicks into a 96-well plate containing 200 µl fermentation medium in each well and cultured at 30°C for 24 h. The rotating speed of the plate shaker was 800 rpm. After completion of fermentation, L-lysine yield was measured. All clones from the library of position 293 mutants had a yield higher than 0.5 g/L; some clones from the library of position 294 mutants and position 307 mutants had a yield higher than 0.5 g/L, indicating that mutants with mutations of the above positions can remove lysine feedback inhibition and increase lysine production. The mutants obtaining a lysine yield higher than 0.5 g/L, and some mutants from the library of position 294 mutants and position 307 mutants which did not produce lysine were subjected to sequencing analysis by PCR amplification of a target band using *lysC-*C1/ *lysC-*C2 (Table 2) primers. The yield of the mutants and the sequencing results were as shown in Table 3. Some clones had identical mutations. Finally, 11 mutants were obtained from position 293 mutants, 6 obtained from position 294 mutants, and 5 obtained from position 307 mutants.

**Table 3**

| Strain number | L-lysine yield | Corresponding codon mutation | Amino acid mutation |
|---|---|---|---|
| 293-M1 | ++++ | TCC | 1293 S |
| 293-M2 | ++++ | GGC | I293 G |
| 293-M3 | ++++ | GAA | I293E |
| 293-M4 | ++++ | CCA | I293P |
| 293-M5 | ++++ | TGG | I293W |
| 293-M6 | ++++ | TAC | 1293 Y |
| 293-M7 | ++++ | CAC | I293H |
| 293-M8 | ++++ | ATG | I293M |
| 293-M9 | ++++ | CAA | I293Q |
| 293-M10 | + | TGC | I293C |
| 293-M11 | ++ | CGC | I293R |
| 294-M1 | + | TAC | D294Y |
| 294-M2 | ++ | TGG | D294W |
| 294-M3 | +++ | TTC | D294F |
| 294-M4 | - | CTC | D294L |
| 294-M5 | - | GTC | D294V |
| 294-M6 | - | TGC | D294C |
| 307-M1 | + | TAC | T307Y |
| 307-M2 | +++ | GGC | T307G |
| 307-M3 | + | TTC | T307F |
| 307-M4 | - | GAA | T307E |
| 307-M5 | - | AAG | T307K |

| | | | |
|---|---|---|---|
| Note: -: no lysine produced; +: lysine yield ranging 0.5to 1.0 g/L; ++: lysine yield ranging 1.0 to 2.0 g/L; +++: lysine yield ranging 2.0 to 3.0 g/L; ++++: lysine yield ranging 3.0 to 4.0 g/L. | | | |

### Example 3. Assessment of lysine production of Corynebacterium glutamicum lysC gene mutants

Since the strains previously used for screening mutants based on lysine yield included pRecT and pCas9gRNA-*lysC*1 or pCas9gRNA-*lysC*2 plasmid, thus in this example, the above plasmids were deleted, and strains that only having mutations in the genome were obtained and the lysine yield of these strains were assessed. The relatively high-yield strain 293-M1 to strain 293-M9, strain 293-M1 to strain 293-M3, strain 307-M1 to strain 307-M2 were cultured in a chloromycetin-free TSB medium, with two plasmids deleted, respectively obtaining mutant strains of ZCgLJ1 to ZCgLJ9, ZCgLJ11 to ZCgLJ13, ZCgLJ14 to ZCgLJ15 with the plasmids deleted. For the purpose of effect comparison, a huge amount of *lysC*^{T311I} mutants reported in prior literature that effectively remove lysine feedback inhibition were constructed at the same time. Such strains were obtained by introducing T311I (i.e. the bases mutated from ACC to ATC) amino acid mutation into the *lysC* gene encoding aspartate kinase of *Corynebacterium glutamicum* ATCC13032 (Reference Document 2). Further, the wild-type *Corynebacterium glutamicum* ATCC13032 was used as the control strain (WT). A 24-well plate was used to assess the lysine production of the strains. The strains were firstly inoculated into the TSB liquid medium and cultured for 6-8. The cultures were inoculated as seeds into a 24-well plate containing 600 µl of fermentation medium in each well (the same as in Example 2) with the initial OD₆₀₀ controlled at about 0.1, and cultured at 30°C for 17 h. The rotating speed of the plate shaker was 800 rpm. Three samples were set for each strain. After the fermentation, the lysine yield was measured. The results were listed in Table 4. All of the 11 mutants with mutation at position 293 had higher yield than the T311I control mutant. D294F (ZCgLJ13 strain) mutants with mutation at position 294 and T307G (ZCgLJ15) mutants with mutation at position 307 also had higher yield than the T311I control mutant. Mutations at these amino acid positions were more promising in application of the production of amino acids and derivatives thereof than T311I, in particular in the production of lysine, threonine and isoleucine and derivatives thereof such as pentanediamine, 5-aminovaleric acid, glutaric acid, hydroxyisoleucine, which all rely on LysC catalyzing reactions.

**Table 4**

| Stain | Amino acid mutation | OD₆₀₀ | lysine yield (g/L) |
|---|---|---|---|
| WT | - | 11.78±0.18 | 0.17±0.06 |
| *lysC*^{T311I} | T311I | 12.15±0.23 | 2.33±0.06 |
| ZCgLJ1 | I293S | 12.50±0.35 | 3.27±0.15 |
| ZCgLJ2 | 1293 G | 11.99±0.34 | 3.00±0.10 |
| ZCgLJ3 | I293E | 11.86±0.59 | 3.00±0.20 |
| ZCgLJ4 | I293P | 12.22±0.55 | 3.30±0.20 |
| ZCgLJ5 | I293W | 12.35±0.03 | 3.33±0.06 |
| ZCgLJ6 | I293Y | 12.72±0.24 | 3.53±0.06 |
| ZCgLJ7 | I293H | 12.40±0.08 | 3.30±0.00 |
| ZCgLJ8 | I293M | 12.15±0.25 | 2.77±0.06 |
| ZCgLJ9 | 1293 Q | 12.23±0.06 | 3.50±0.00 |
| ZCgLJ11 | D294Y | 12.00±0.51 | 0.60±0.00 |
| ZCgLJ12 | D294W | 12.04±0.41 | 1.73±0.06 |
| ZCgLJ13 | D294F | 12.66±0.20 | 2.73±0.06 |
| ZCgLJ14 | T307Y | 12.14±0.41 | 0.7±0.00 |
| ZCgLJ15 | T307G | 12.41±0.18 | 2.50±0.00 |

All technical features disclosed herein are able to be combined to form any combination. Each feature disclosed herein may be substituted by a feature having an identical, equivalent, or similar function. Therefore, unless otherwise specified, each feature disclosed herein is merely a practical example of a series of equivalent or similar features.

In addition, based on the foregoing description of the present disclosure, a person skilled in the art clearly knows the key features of the present disclosure. Without departing from the spirits and scopes of the present disclosure, a number of alterations may be made to the invention to adapt to different purposes and conditions of use. Therefore, it is intended that such alterations also fall in the scope of attached claims.

Reference:
[1] Bearer CF, Neet KE; Stadtman, E. R., Cohen, G. N., LeBras, G., Robichon-Szulmajster, H. (1961). "Feed-back Inhibition and Repression of Aspartokinase Activity in Escherichia coli and Saccharomyces cerevisiae". J. Biol. Chem.
[2] Watson et. al. (1987) Molecular Biology of the Gene. Fourth Edition. Benjamin/Cummings Pub. Co. P224.
[3] LIU, Jiao, et al. "Development of a CRISPR/Cas9 genome editing toolbox for Corynebacterium glutamicum". Microbial cell factories, 2017, 16.1: 205.
[4] WANG, Yu, et al. "Expanding targeting scope, editing window, and base transition capability of base editing in Corynebacterium glutamicum". Biotechnology and bioengineering, 2019, 116: 3016-3029.
[5] Ruan Y, Zhu L, Li Q. "Improving the electro-transformation efficiency of Corynebacterium glutamicum by weakening its cell wall and increasing the cytoplasmic membrane fluidity". Biotechnol Lett. 2015; 37: 2445-52.

## Claims

1. A polypeptide with aspartate kinase activity, wherein the polypeptide is selected from a group consisting of any one of (i) to (iv):
(i) a mutant of the polypeptide having the sequence as shown in SEQ ID NO: 1, compared with the sequence as shown in SEQ ID NO: 1, the mutant comprising a mutation at least at one or more positions corresponding to positions 293, 294, and 307 of the sequence as shown in SEQ ID NO: 1;
(ii) a polypeptide having at least 70%, at least 80%, or at least 90% sequence identity with the sequence as shown in (i) and not comprising the polypeptide having the sequence as shown in SEQ ID NO: 1;
(iii) a polypeptide encoded by a polynucleotide, the polynucleotide being hybridized with the polynucleotide as shown in (a) or (b) under very high stringent conditions:
(a) a polynucleotide encoding a polypeptide having the amino acid sequence as shown in (i);
(b) a full-length complementary polynucleotide of (a);
(iv) a fragment of the polypeptide as shown in (i), (ii), (iii), the fragment still having aspartate kinase activity.

2. The polypeptide according to claim 1, wherein the polypeptide is a polypeptide comprising a mutation as shown in at least one group of (c) to (e) as follows:
(c) the amino acid at a position corresponding to position 293 of the sequence as shown in SEQ ID NO: 1 being mutated from isoleucine (I) to serine (S), glycine (G), glutamic acid (E), proline (P), tryptophan (W), tyrosine (Y), histidine (H), methionine (M), glutamine (Q), cysteine (C), or arginine (R);
(d) the amino acid at a position corresponding to position 294 of the sequence as shown in SEQ ID NO: 1 being mutated from aspartic acid (D) to tyrosine (Y), tryptophan (W), or phenylalanine (F);
(e) the amino acid at a position corresponding to position 307 of the sequence as shown in SEQ ID NO: 1 being mutated from threonine (T) to tyrosine (Y), glycine (G), or phenylalanine (F).

3. The polypeptide according to claim 1 or 2, wherein the polypeptide comprises deletion or addition at least one amino acid residue at N-terminus or C-terminus of the polypeptide having the sequence as shown in (i).

4. A recombinant polypeptide comprising the polypeptide according to any one of claims 1 to 3 and an exogenous polypeptide fused to the polypeptide; optionally, the exogenous polypeptide comprising a tag polypeptide; preferably, the exogenous polypeptide comprising a tag polypeptide and a spacer polypeptide linking the tag polypeptide to the polypeptide with aspartate kinase activity.

5. An isolated polynucleotide, comprising a nucleotide sequence encoding the polypeptide according to any one of claims 1 to 3, or comprising a nucleotide sequence encoding the recombinant polypeptide according to claim 4.

6. A nucleic acid construct, comprising the polynucleotide according to claim 5, the polynucleotide is operably linked to one or more regulatory sequence, the regulatory sequence directs production of polypeptides in an expression host.

7. A recombinant expression vector, comprising the polynucleotide according to claim 5, or the nucleic acid construct according to claim 5.

8. A recombinant host cell, comprising the polypeptide according to any one of claims 1 to 3, the recombinant polypeptide according to claim 4, the polynucleotide according to claim 5, the nucleic acid construct according to claim 6, or the recombinant expression vector according to claim 7.

9. The recombinant host cell according to claim 8, wherein the host cell is from genus *Corynebacterium,* genus *Brevibacterium,* genus *Arthrobacterium,* genus *Microbacterium,* or genus *Escherichia*; preferably, the host cell is *Corynebacterium glutamicum* or *Escherichia coli.*

10. Use of the polypeptide according to any one of claims 1 to 3, the recombinant polypeptide according to claim 4, the polynucleotide according to claim 5, the nucleic acid construct according to claim 6, the recombinant expression vector according to claim 7, or the recombinant host cell according to claim 8 or 9 in production of an amino acid;
preferably, the amino acid being selected from lysine, threonine, isoleucine and an amino acid derivative thereof, wherein the amino acid derivative thereof includes at least one of pentanediamine, 5-aminovaleric acid, glutaric acid, and hydroxyisoleucine.

11. A method for producing an amino acid, comprising steps of producing an amino acid using the polypeptide according to any one of claims 1 to 3, the recombinant polypeptide according to claim 4, the polynucleotide according to claim 5, the nucleic acid construct according to claim 6, the recombinant expression vector according to claim 7, or the recombinant host cell according to claim 8 or 9;
optionally, the method further comprising a step of purifying or isolating the amino acid;
preferably, the amino acid being selected from lysine, threonine, isoleucine and an amino acid derivative thereof, wherein the amino acid derivative thereof includes at least one of pentanediamine, 5-aminovaleric acid, glutaric acid, and hydroxyisoleucine.
